Europäisches Patentamt

European Patent Office

Office européen des brevets

(11) Veröffentlichungsnummer: **0 349 520**
A2

# (12) EUROPÄISCHE PATENTANMELDUNG

(21) Anmeldenummer: 89890119.4

(22) Anmeldetag: 25.04.89

(51) Int. Cl.⁵: **G 01 N 33/542**
G 01 N 33/58, G 01 N 33/543,
G 01 N 21/64

(30) Priorität: 28.06.88 AT 1671/88

(43) Veröffentlichungstag der Anmeldung:
03.01.90 Patentblatt 90/01

(84) Benannte Vertragsstaaten:
**BE CH DE FR GB IT LI NL SE**

(71) Anmelder: **Koller, Ernst, Dr.**
**A-8082 Kirchbach 111a (AT)**

**Wolfbeis, Otto S., Dr.**
**Im Hoffeld 32**
**A-8046 Graz (AT)**

(72) Erfinder: **Koller, Ernst, Dr.**
**A-8082 Kirchbach 111a (AT)**

**Wolfbeis, Otto S., Dr.**
**Im Hoffeld 32**
**A-8046 Graz (AT)**

(74) Vertreter: **Pinter, Rudolf**
**Patentanwalt Dipl.-Ing. Rudolf Pinter Elisabethstrasse 1/24**
**A-1010 Wien (AT)**

(54) **Fluoreszenz-Immunoassay auf Grundlage der Fluoreszenzlöschung.**

(57) Der Fluoreszenzimmunoassay beruht auf dem Befund, daß die Fluoreszenz eines Antikörpers oder Antigens durch einen der Analysenlösung zugesetzten Fluoreszenzlöscher stärker gelöscht wird, solange sich der Immunkomplex noch nicht gebildet hat. Durch Bestimmung der Löscheffizienz vor und nach der Komplexbildung kann man auf die Konzentration des entsprechenden Antigens oder Antikörpers schließen.

Fig. 5

EP 0 349 520 A2

**Beschreibung**

## FLUORESZENZ-IMMUNOASSAY AUF GRUNDLAGE DER FLUORESZENZLÖSCHUNG

Die vorliegende Erfindung betrifft ein Verfahren zum Fluoreszenz-Immunoassay, bei welchem das Ausmaß der Löschung der Fluoreszenz eines Antigens oder Antikörpers durch einen extern zugesetzten Löscher vor und nach erfolgter Komplexbildung gemessen wird, und bei dem aus der Änderung der Löscheffizienz als Folge des Bindungsprozesses auf die Konzentration von Antigen bzw. Antikörper in einer unbekannten Probe geschlossen werden kann.

Immunoassaymethoden haben sich als äußerst nützlich für die qualitative und quantitative Bestimmung biologischer Substanzen herausgestellt, da immunologische Reaktionen mit hoher Spezifität ablaufen und mit hoher Sensitivität detektiert werden können. In letzter Zeit wurde aber der klassische Radioimmunoassay (RIA) in zunehmendem Maß durch den Fluoreszenzimmunoassay (FIA) ersetzt, da der Umgang mit radioaktiv markierten Substanzen mit verschiedenen Nachteilen behaftet ist. Obwohl FIA-Methoden meist nicht so empfindlich sind wie der RIA, haben sie eine stürmische Entwicklung erfahren, da die Fluoreszenzspektrometrie zu den vielseitigsten Methoden der Analytik gehört. Folgende Übersichtsartikel sind repräsentativ:

G. C. Visor und S. G. Schulman, J. Pharm. Sci. 70, 469 (1981);
R. P. Ekins und S. Dakubu, "The Development of High Sensitivity Pulsed-Light, Time-Resolved FIA", Pure & Appl. Chem. 57, 473 (1985);
T. S. Smith, M. Hassan und R. D. Nargessy, "Principles and Practice of FIA Procedures", in: Modern Fluorescence Spectroscopy, vol. 3 (E. L. Wehry, editor), Plenum Press, New York, 1981;
N. J. Seare, "Immunoassay Techniques", in: Chemical Sensors (T. E. Edmonds, editor), Chapman and Hall, New York, 1988;
I. Karube, "Novel Immunosensors", in Biosensors 2, 343 (1986).
J. S. Woodhead und I. Weeks, "Chemiluminescence Immunoassay", Pure & Appl. Chem. 57, 523 (1985).
M. J. Grayeski, "Chemiluminescence Immunoassay", Anal. Chem. 59, 1250A (1987).

In all diesen Verfahren wird ein Antikörper (Ab) oder ein Antigen (Ag) mit einem fluoreszierenden Molekül markiert und die Änderung der Fluoreszenzeigenschaften des Fluoreszenzmarkers als Folge der Bindung des Antikörpers an das Antigen untersucht. Man unterscheidet dabei zwischen homogenem und heterogenem FIA. Die homogenen FIAs besitzen den Vorteil, daß kein Trennschritt notwendig ist, um gebundenes von ungebundenem Protein zu trennen. Man unterscheidet weiters je nach Art der spektroskopischen Methode zwischen mehreren Typen von FIAs:

1) FIA auf der Grundlage von Intensitätsmessungen
Diese Methoden beruhen auf einer beobachteten Reduktion oder einer Zunahme in der Fluoreszenzintensität eines markierten Liganden nach der Bindung durch den Antikörper. Die Ursache für diese Intensitätsänderung ist nicht genau bekannt, aber man nimmt an, daß es sich um eine Änderung der elektronischen Struktur des Fluoreszenzfarbstoffes handelt. Veränderungen der elektronischen Verteilung des gebundenen, markierten Liganden können die strahlungslose Desaktivierung von angeregten Molekülen verstärken. Als eine andere Interpretation wird die Möglichkeit ins Auge gefaßt, daß die Polarität der Umgebung des Fluoreszenzmarkers durch die Bindung so verändert wird, daß sich die Quantenausbeute des Fluorophors charakteristisch ändert (Solvatochromie). Es kann dabei sowohl zu einer Zunahme als auch zu einer Abnahme der Fluoreszenz kommen. Eine Voraussage, in welche Richtung diese Änderung gehen soll, ist nicht möglich.

2) Energietransfer - FIA
Wenn die Absorptionsbande eines Fluorophors (genannt Akzeptor) mit der Fluoreszenz eines anderen Fluorophors (genannt Donor) überlappt, kann es zu einem elektronischen Energietransfer (ET) vom Donor zum Akzeptor kommen. Bekanntestes Beispiel ist das Paar Fluorescein und Rhodamin. Regt man in einem Gemisch der beiden das Fluorescein an, so beobachtet man die Fluoreszenz des Rhodamins. Die Effizienz des ET hängt umgekehrt von der 6. Potenz der Entfernung der beiden Fluorophore ab. Solange also z. B. das Antigen (Rhodamin-markiert) und der Antikörper (Fluorescein-markiert) noch nicht aneinander gebunden vorliegen, findet kein ET statt. Sind sie gebunden, wird ET beobachtet. Ein typisches Anwendungsbeispiel wird von Fisher et al. in Clin. Chem. 26, 987 (1980) beschrie ben.

In einer anderen Form kann der Energieakzeptor auch nicht fluoreszierend sein, solange nur sein Absorptionsspektrum mit dem Emissionsspektrum des Donors übereinstimmt. Diese Methode ist genauso umständlich wie die vorherige, da sie die Markierung sowohl von Ab als auch von Ag erfordern. Ein typisches Beispiel ist beschrieben von Velich et al. in Proc. Natl. Acad. Sci. 46, 1470 (1969).

3) Fluoreszenzpolarisation
Während die Fluoreszenzemission eines kleinen und schnell rotierenden Moleküls praktisch nicht polarisiert ist, weil sich während der Lebenszeit des angeregten Zustandes alle Moleküle in statistischer Weise drehen und so nach allen Richtungen und in allen Ebenen emittieren, ist dies bei einem Komplex aus Antigen und Antikörper nicht mehr der Fall, da es sich dabei um sehr große Moleküle mit kleiner Rotationsgeschwindigkeit handelt. Man beobachtet also, daß der Grad an Fluoreszenzpolarisation in dem Maß ansteigt, in dem ein fluoreszenzmarkierter Antikörper durch ein Antigen gebunden wird, bzw. umgekehrt. Der Polarisationsgrad P kann Werte zwischen +1/2 und -1/3 annehmen. Aus dem Umstand, daß P in Abhängigkeit vom Ausmaß der Bindung eines markierten Liganden variiert, kann man durch Auftragung von P gegen die Konzentration des unmarkierten Liganden eine Eichkurve aufstellen, mit deren Hilfe ein homogener Immunoassay durchgeführt werden kann. Ein typisches Beispiel ist beschrieben von Lu-Steffes et al. in Clin. Chem. 28, 2278 (1982). Das Prinzip wird in verschiedenen kommerziell erhältlichen Geräten angewendet. In US-Patent 4.451.149 wird eine solche Methode in Kombination mit faseroptischen Lichtleitern beschrieben.

4) Zeitaufgelöste FIA

So wie sich die Fluoreszenzintensität bzw. -polarisation eines Fluorophors durch die Bildung eines Ag/Ab-Komplexes ändert, ändert sich auch die Fluoreszenzabklingzeit. Durch die Messung der Änderung der Abklingzeit des Fluoreszenzmarkers als Folge des Bindungsvorganges kann auf die Menge von Antigen geschlossen werden. Ein typisches Beispiel ist bekannt aus der DE-OS 2.628.158, wo ein Fluoreszenzmarker mit besonders langer Abklingzeit eingesetzt wird. Eine detaillierte Beschreibung findet sich in einem Artikel von Hemmila et al. in Anal. Biochem. 137, 335 (1984).

Die Methode hat den Vorteil, daß sie Untergrundstrahlung (z. B. durch Raman- und Rayleigh-Streuung) gut unterdrücken kann. Sie erfordert aber eine aufwendige elektronische Konfiguration, um die Abklingzeit des Komplexes von jener des freien Liganden zu trennen, und die Verwendung verschiedener zusätzlicher Reagentiensätze.

5) Enzymatische FIAs

Wird ein Enzym an einen Antikörper gebunden, so ist es wie in Lösung in der Lage, Enzymsubstrate zu spalten. Aus nichtfluoreszierenden Substraten entstehen so fluoreszierende Hydrolyseprodukte. Wird aber der Ab/Ag-Komplex gebildet, ist das Enzym für das Substrat nicht mehr zugänglich, und dieses kann nicht mehr so schnell gespalten werden. Die verminderte Hydrolysegeschwindigkeit ist somit ein Maß für die Menge an gebildetem Ab/Ag-Komplex. Eine typische Anwendung ist die Bestimmung von IgM, beschrieben von Warah et al. in Clin. Chem. 27, 673 (1981).

Ein relativ umständlicher Immunoassay auf Grundlage der Fluoreszenzlöschung ist in der Eur. Appl. EP 104,926 beschrieben: Danach wird zur Detektion einer immunogenen Substanz wie z.B. eines Medikamentes dieses zuerst an ein großes Protein gebunden, dann ein entsprechender Antikörper isoliert, und dieses zusammen mit einem fluoreszenzmarkiertem Hapten im Immunoassay eingesetzt. Das Verfahren ist zeitaufwendig und arbeitsintensiv.

Neben den bisher besprochenen Methoden gibt es noch heterogene FIAs, welche durch erhöhten Aufwand und bessere Sensitivität gekennzeichnet sind. Bei diesen Verfahren wird der Antikörper-Antigen-Komplex zuerst von den freien Liganden abgetrennt und dann erst einer Quantifizierung zugeführt. Der Vorteil besteht darin, daß damit die Untergrundfluoreszenz praktisch vollständig eliminiert wird, wodurch es zu einer beträchtlichen Erhöhung der Empfindlichkeit kommt.

Die vorliegende Erfindung beruht auf dem Prinzip der Fluoreszenzlöschung durch einen extern zugesetzten Löscher. Es ist bekannt, daß verschiedene Fluorophore auf Zusatz von sogenannten Löscher-Molekülen in ihrer Fluoreszenzintensität geschwächt werden. Dieses je nach Mechanismus als statische oder dynamische Fluoreszenzlöschung bezeichnete Phänomen beruht darauf, daß der zugefügte Löscher die Quantenausbeute und, im Fall der dynamischen Löschung auch die mittlere Lebenszeit des Fluorophors verringern kann.

Ursache für die statische Löschung ist die Bildung eines nichtfluoreszierenden Fluorophor-Löscher-Komplexes im elektronischen Grundzustand. Ursache für die dynamische Löschung ist meist ein Elektronentransfer im ersten angeregten Zustand, welcher zur Bildung eines nichtfluoreszierenden Moleküls führt. Typische Fluorophor-Löscher-Kombinationen sind in der folgenden Tabelle zusammengestellt.

## Tab. 1.

Typische Fluorophor-Löscher-Kombinationen und Stern-Volmer-Löschkonstanten in Wasser bei Raumtemperatur.

| Fluorophor | Löscher | K |
|---|---|---|
| Chinin-Kation | Chlorid | 115 M$^{-1}$ |
| N-Methylacridinium | Bromid, Iodid | 290, 385 M$^{-1}$ |
| 6-Methoxychinolinium | Iodid, Ag(I), Resorcin | 255, - M$^{-1}$ |
| Pyren | Sauerstoff | 0.08, Torr$^{-1}$ |
| Anthracene | O$_2$, SO$_2$, (Sulfit) | 0.05, 0.010 Torr$^{-1}$ |
| Perylen | Ag(I), Pb(II) | - |
| Fluorscein, Rhodamin | Iodid, Azid | ca. 170, 7 M$^{-1}$ |
| Tryptophan | Pyridin, Acrylamid | ca. 6x10$^{-4}$ M$^{-1}$ |
| Tryptophan | Bromid, Iodid | - |
| Porphyrin | Nitroaromaten | - |
| Heterocyclen | Azide, Isocyanate | 20-330 M$^{-1}$ |
| Indole, Carbazole | Pyridin | ca. 10$^5$ M$^{-1}$ |
| Chinin | Nicotinamid | - |
| APTS[a] | Thiamin (Vitamin B$_1$) | ca. 445 M$^{-1}$ |
| 9-Aminoacridin | versch. Purine | - |
| Flavin | Adenin | - |
| AOPTS[b] | kationische Detergentien | - |

[a] 1-Aminopyren-3,6,8-trisulfonat,
[b] 1-Alkoxypyren-3,6,8-trisulfonat

Nach Stern und Volmer besteht zwischen der Fluoreszenzintensität des Fluorophors in Gegenwart bzw. Abwesenheit eines Löschers und der Konzentration am Löscher die folgende Beziehung

$$I_o/I = 1 + K\ [Q] \qquad (1)$$

worin $I_o$ und $I$ die Fluoreszenzintensität des Fluorophors in Abwesenheit bzw. Gegenwart eines Löschers bedeuten, welcher in einer Konzentration [Q] vorhanden ist. K ist die sogenannte Lösch-konstante, welche für jedes Paar von Fluorophor und Löscher spezifisch ist und unter anderem von der Temperatur, der Viskosität und dem Lösungmittel abhängt.

Anstelle der Intensität I kann im Fall der dynamischen Löschung auch die Lebensdauer des Fluorophors herangezogen werden, da auch

$$t_o/t = 1 + K\ [Q] \qquad (2)$$

$t_o$ und $t$ sind die Abklingzeiten des Fluorophors in Abwesenheit bzw. Gegenwart eines Löschers in Konzentration [Q].

Der erfindungsgemäße FIA beruht darauf, daß man die Löschung der Fluoreszenz eines Antikörpers oder eines Antigens durch einen externen Löscher vor und nach der Bildung des Ag/Ab-Komplexes untersucht. Man kann dabei zwischen zwei Arten von fluoreszierenden Antikörpern und Antigenen unterscheiden: Einerseits solche, welche eine Eigenfluoreszenz aufweisen, andrerseits solche, welche mit einem Fluorophor markiert sind. Eigenfluoreszenz wird dann beobachtet, wenn sich im Molekül Tyrosin- (Tyr) oder Tryptophanbausteine (Trp) befinden. Die Fluoreszenz kann dann bei 280 bzw. 295 nm angeregt werden und hat ihr Maximum bei 300 bis 350 nm. Häufiger ist der zweite Fall, bei dem ein Antigen oder Antikörper mit einem synthetischen Fluorophor markiert wird.

Verfahren zur Markierung von Proteinen bzw. Antigen und Antikörpern mit fluoreszierenden Markern sind als Stand der Kunst anzusehen. Eine Beschreibung der entsprechenden Methoden findet sich in: R. P. Haugland, "Covalent Fluorescent Probes" im Buch von R. F. Steiner: "Excited States of Biopolymers", Plenum Press, New York, 1983. Am gängigsten sind Umsetzungen mit fluoreszierendem Isothiocyanaten (welche mit den endständigen Aminogruppen der Proteine reagieren), mit Sulfonsäurechloriden (welche mit Aminen und Hydroxygruppen reagieren), sowie mit Iodacetami den und Maleinimiden (welche mit SH-Gruppen zur Reaktion gebracht werden können).

Ein wesentliches Kennzeichen der erfindungsgemäßen Methode ist der Umstand, daß sich der Löscher im Lösungsmittel befindet und entwedernicht fluoreszierend oder höchstens nur schwach fluoreszierend (wie etwa im Fall des Resorcins) ist. Wenn er fluoreszierend ist, so liegt aber seine Fluoreszenz so, daß es nicht zu einem Energietransfer kommen kann. Typische Löschermoleküle sind in Tab. 1 aufgeführt, ohne damit aber den Anspruch auf Vollständigkeit zu erheben. Man unterscheidet zwischen ionischen Löschern wie z. B. den Halogeniden, und neutralen Löschern wie z. B. Sauerstoff, Acrylamid, Pyridin und Resorcin. Solange sich nun der eine Bindungspartner frei in Lösung befindet, wird seine Fluoreszenz durch die im Lösungmittel anwesenden Löschermoleküle stark erniedrigt. Nach der Bildung des Ag/Ab-Komplexes hingegen wird der Fluorophor von den Löschermolekülen sterisch abgeschirmt, sodaß es zu einer geringeren Anzahl von Kollisionen (und damit Löschvorgängen) kommt. Als Ergebnis beobachtet man einen Anstieg in der Fluoreszenzintensität bzw. der Abklingzeit als Folge des Bindungsprozesses.

Die folgenden Abbildungen sollen die Erfindung erläutern und ihre Vorteile gegenüber bisherigen Methoden herausstellen.

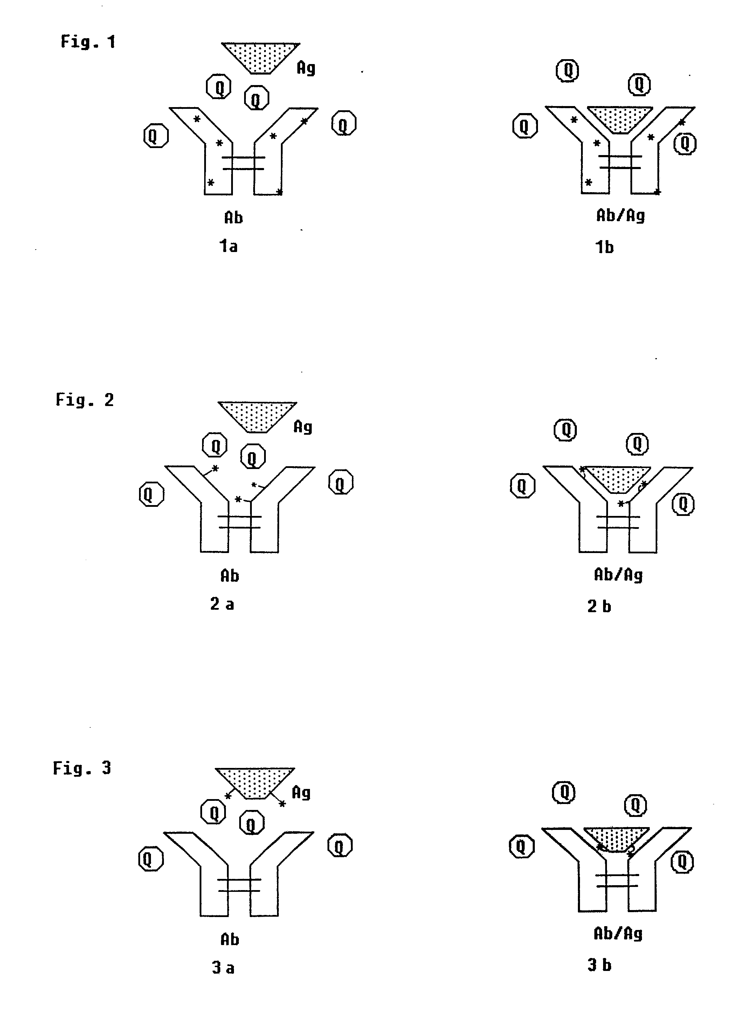

Fig. 1 zeigt schematisch einen typischen Y-förmigen Antikörper (Ab) vor (1a) und nach der Bindung (1b) an ein Antigen (Ag). Es ist offensichtlich, daß ein in Lösung vorhandener Quencher (Q) das im Protein enthaltene fluoreszierende Tyrosin oder Tryptophan (gekennzeichnet durch einen Stern) mit viel größerer Effizienz zu löschen vermag, solange das Antigen noch nicht gebunden ist. Der Vorteil der Methode besteht darin, daß das Ab oder Ag nicht markiert zu werden braucht und daß man den Löscher in genau definierter Konzentration bereits dem Lösungsmittel zugeben kann.

Fig. 2 zeigt schematisch einen fluoreszenz-markierten Antikörper (Ab) vor (2a) und nach der Bindung (2b) an ein Antigen (Ag). Der Fluorophor, gekennzeichnet durch einen Stern, ist kovalent an den Antikörper gebunden. Wieder ist offensichtlich, daß ein in Lösung vorhandener Quencher (Q) den Fluorophor mit viel größerer Effizienz zu löschen vermag, solange das Antigen noch nicht gebunden ist. Der Vorteil dieser Methode besteht darin, daß nur das Ab oder Ag markiert zu werden braucht und daß man den Löscher in genau definierter Konzentration bereits dem Lösungsmittel (und nicht etwa dem anderen Bindungspartner) zugeben kann.

Fig. 3 zeigt die entsprechende Darstellung eines fluoreszenz-markierten Antigens vor (3a) und nach der Bindung (3b) an einen Antikörper.

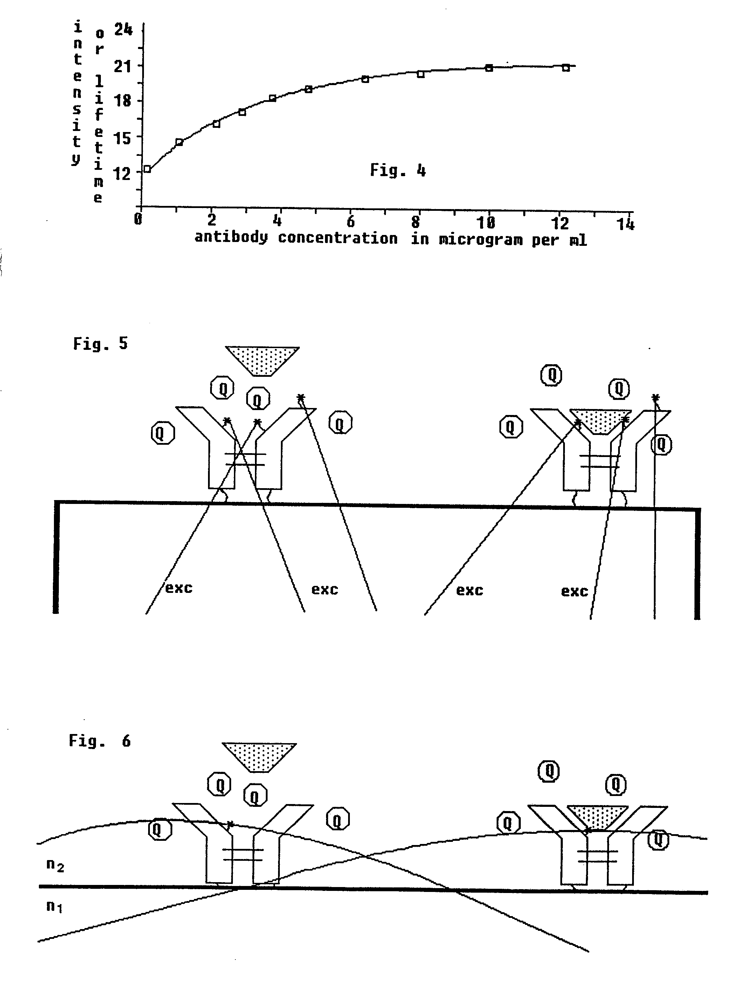

Fig. 4 zeigt eine typische Kurve, wie sie aus der Auftragung von relativer Fluoreszenzintensität [I in Gl. (1)] oder relativer Abklingzeit [t in Gl. (2), angegeben in nano-Sekunden] eines Antigens gegen eine zugesetzte Menge von Antikörper erhalten wurde. Die Messung reduziert sich also auf eine Intensitäts- oder Abklingzeitbestimmung. Es sind keine Polarisatoren erforderlich, und eine Trennung des Ab/Ag-Komplexes vom Rest der Probe kann (muß aber nicht) entfallen.

Fig. 5 zeigt schematisch eine experimentelle Anordnung zur Messung der Fluoreszenzintensität eines auf einem optisch transparenten Träger oder Ende eines faseroptischen Lichtleiters immobilisierten Antigens oder Antikörpers. Der Antikörper kann am distalen oder seitlichen Ende des Lichtleiters entweder direkt immobilisiert werden, oder er wird zuerst auf einem Träger (z. B. einer Polymermembran) angebracht und dieser wird dann am Ende des Lichtleiters fixiert. Solche Trägermembranen sind aus Plastikmaterialien sehr billig herzustellen. Somit können Tests auch mit Hilfe einmal verwendbarer und wegwerfbarer Trägermaterialien durchgeführt werden.

Mit Hilfe der faseroptischen Lichtleiter ist es schließlich auch möglich, derartige immunologische Messungen auch invasiv durchzuführen.

Fig. 6 zeigt schließlich eine Anordnung zur Messung der Fluoreszenz eines markierten Ab oder Ag mit Hilfe der internen Totalreflexion. Es ist bekannt, daß Licht bei der Totalreflexion an einer Grenzfläche zweier Medien mit Brechungsindex $n_1$ und $n_2$ nicht direkt an der Grenzfläche reflektiert wird, sondern daß das elektromagnetische Feld einige nm in die zweite Phase penetriert ("evanesziert"). Es kann dort Fluoreszenz erregen, welche vom Lichtwellenleiter fortgeleitet werden kann. Der Vorteil dieser Methode besteht darin, daß man eine sehr definierte Eindringtiefe hat (welche von den Brechungsindices und der Wellenlänge des Lichtes abhängt), sodaß sich über der Probe (in diesem Fall ein Ag oder ein Ab) eine optisch nicht transparente Phase (z. B. Blut) befinden kann, ohne daß dadurch eine Störung eintritt.

Ein typisches Verfahren sei nun an der Bestimmung von Immunoglobulin G (IgG) im Serum erläutert. Man verwendet dazu z. B. Anti-Human IgG (aus Schafen), welches mit Fluorescein-isothiocyanat (FITC) in 4 ml Bicarbonatpuffer von pH 9,5 für 24 Std. bei 22° C umgesetzt wird (50 mg IgG und 4 mg FITC). Das FITC-markierte Protein wird vom unumgesetzten FITC an einer Sephadex G-25 Kolonne (10 cm x 2 cm) abgetrennt. Das markierte Protein wird in einer Konzentration von typischerweise 0,1 - 1,0 mg/1 in einer 0,1 normalen Lösung von Kaliumbromid in Wasser mit 0,1% Emulgatorzusatz gelöst. Anstelle von Natriumbromid kann auch Natriumiodid oder -azid verwendet werden.

Iodid, Azid bzw. Bromid sind starke Löscher der Fluoreszenz von FITC. Solange das oberflächlich an Anti-IgG gebundene Fluorescein der freien Lösung ausgesetzt ist, wird es durch die anwesenden Halogenidionen in seiner Fluoreszenzintensität stark gelöscht bzw. in seiner Fluoreszenzabklingzeit entsprechend Gleichung (2) vermindert.

Setzt man aber dieser Lösung zunehmende Mengen an Serum zu, welches IgG enthält, so wird sich ein Komplex aus IgG und Anti-IgG bilden, wodurch das FITC des Antikörpers von der Lösung abgeschirmt wird, sodaß es in seiner Fluoreszenz weniger leicht gelöscht wird. Bis zu jenem Punkt, an welchem alles Anti-IgG gebunden ist, beobachtet man also eine kontinuierliche Zunahme der Fluoreszenz. Diese setzt sich zusammen aus (a) großteils gelöschter Fluoreszenz des ungebundenen FITC/Anti-IgG und (b) praktisch ungelöschter Fluoreszenz des FITC im Antikörper-Antigen-Komplex.

Es resultiert eine Kalibrationskurve, wie sie in Fig. 4 dargestellt ist. Durch Vergleich der Fluoreszenzintensität der reinen FITC-Anti-IgG-Lösung mit der Fluoreszenzintensiät der mit Serum versetzten Lösung hat man an Hand der Eichkurve die Möglichkeit, eine quantitative Bestimmung von IgG durchzuführen.

Neben dem in diesem Beispiel verwendeten FITC kommen eine Reihe von anderen Fluorophoren als Marker in Frage. Besonders erwähnenswert ist die relativ spezifische Löschung von Acridinium-Ion und 6-Methoxychinolinium-Ion durch Chlorid, Bromid und Iodid. Der Löscher Chlorid kommt im Humanblut in einer Konzentration von ca. 100 mMol/l vor, sodaß Chloridion bei in-vivo Messungen als Löscher benutzt werden kann.

Die Löschung der intrinsischen Fluoreszenz der Proteine (zurückzuführen auf die Anwesenheit von Tyr und Trp) ist beispielsweise mit Pyridin, Acrylamid, Bromid, Iodid oder Schwermetallsalzen möglich.

In einer besonderen Ausführungsform des Verfahrens wird das markierte Antigen oder der markierte Antikörper an der Oberfläche eines optisch transparenten polymeren Trägers immobilisiert, z. B. an der Oberfläche eines Glasplättchens oder eines faseroptischen Lichtleiters. Man verfolgt die Fluoreszenz bzw. deren Änderungen durch den optisch transparenten Träger, z. B. auch mit Hilfe der evaneszierenden Wellen bei der totalen internen Reflexionsspektroskopie (Fig. 5). Solche optische Methoden sind beschrieben von J. F. Place et al. in einem Artikel "Opto-Electronic Immunosensors: A Review of Optical Immunoassays at Continuous Surfaces" in Biosensors, 1, 321 ff (1985) sowie in der US-PS 4.447.546, worin eine Methode zur immunologischen Bestimmung in einem sehr definierten Probenvolumen mit Hilfe der evaneszierenden Technik beschrieben wird. Die Erfinder geben dort aber die Möglichkeit der dynamischen Fluoreszenzlöschung nicht an und erkennen auch nicht deren spezielle Vorteile gerade in diesem Fall. Ein verwandtes Prinzip ist in der US-PS 4.582.809 beschrieben.

Auch bei Verwendung von Lichtleitern verfolgt man die Änderung des Fluoreszenzsignals als Folge der verhinderten Löschung durch einen extern zugefügten Löscher nach erfolgtem Bindungsprozess. Der Vorteil des Verfahrens mit Hilfe evaneszierender Wellen besteht darin, daß man auch stark gefärbte Lösungen, wie z. B. Blut, untersuchen kann, da die evaneszierende Welle bei der internen Totalreflexion an einer Grenzfläche nur wenige Nanometer in die Proteinphase penetriert. Dadurch wird eine recht selektive Anregung der Fluoreszenz des Tyr oder Trp im Ab oder Ag ermöglicht, und die Untergrundfluoreszenz des biologischen Probenmaterials kann klein gehalten werden.

Wird der Antikörper oder das Antigen an das Ende eines faseroptischen Lichtleiters immobilisiert, so hat man unter Ausnützung des Umstandes, daß sich im Blut verschiedene Fluoreszenzlöscher befinden (z. B. Chlorid), die Möglichkeit in der Hand, immunologische Tests mit einem faseroptischen Katheter direkt im Blut vornehmen zu können. Eine Zusammenstellung der wichtigsten immunologischen Methoden mit Hilfe von Lichtwellenleitern findet sich im zitierten Artikel von J. F. Place et al.

Als extern zugesetzte Löscher kommen vor allem solche in Frage, welche in genau definierter Konzentration in die Meßlösung einzubringen sind. Das sind z. B. die Halogenide und Pseudohalogenide, Pyridine, Acrylamid, Hydrochinon, sowie Übergangsmetall-Kationen, vorzugsweise solche, welche keine Eigenfarbe aufweisen. Auch molekularer Sauerstoff und Schwefeldioxid (bzw. Sulfit) sind bekannt starke Löscher der Fluoreszenz, etwa von aromatischen Kohlenwasserstoffen, doch ist eine definierte Konzentration von z. B. Sauerstoff in wässriger Lösung viel schwieriger herzustellen, da seine Löslichkeit mit dem Proteingehalt variieren kann.

Im Gegensatz zu den bei den Energietransfer-Methoden unter 2) beschriebenen FIAs kann bei der erfindungsgemäßen Methode die Doppelmarkierung entfallen, und es können beliebige Löscher eingesetzt werden, unabhängig davon, ob ihr Absorptionsspektrum mit der Fluoreszenz des ersten Markers übereinstimmt. Auch der Löschmechanismus ist ein ganz anderer, was auch durch die unterschiedliche mathematische Behandlung der beiden Situationen belegt wird: Energietransfer-Löschung erfolgt nach der Theorie von Förster mit einer Effizienz, welche von $R^{-6}$ abhängt (R ist der Abstand der beiden Spezies). Fluoreszenzlöschung durch zugesetzte Löscher gehorcht der Stern-Volmer Gleichung (Gl. 1).

## Patentansprüche

1. Verfahren zur Durchführung eines fluoreszenzoptischen Immunoassays, dadurch gekennzeichnet, daß man

(a) die durch einen in definierter Konzentration zugesetzten externen Löscher verminderte Fluoreszenzintensität oder Fluoreszenzabklingzeit eines gegebenenfalls Antigens oder Antikörpers vorab bestimmt;

(b) den Antikörper bzw. das Antigen in homogener Lösung oder in immobilisierter Form mit dem entsprechenden Antigen bzw. Antikörper der zu untersuchenden Probe zur Reaktion bringt;

(c) das Ausmaß der Löschung der Fluoreszenz des Fluorophors durch den extern zugegebenen Löscher nach der Bindungsreaktion ermittelt; und

(d) mit Hilfe von vorab berechneten bzw. empirisch ermittelten Kalibrationskurven die Menge des in der unbekannten Probe vorhandenen Antigens bzw. Antikörpers ermittelt.

2. Verfahren nach Anspruch 1, dadurch gekennzeichnet, daß der löschbare Fluorophor das Tyrosin oder Tryptophan im Antigen- oder Antikörpermolekül ist.

3. Verfahren nach Anspruch 1, dadurch gekennzeichnet, daß der Fluorophor ein durch kovalente Immobilisierung am Antigen oder Antikörper angebrachter löschbarer Fluoreszent ist.

4. Verfahren nach Anspruch 1, dadurch gekennzeichnet, daß der löschbare Fluorophor durch Halogenide oder Pseudohalogenide gelöscht wird und daß der dem Lösungmittel zugesetzte externe Löscher ein Halogenid- oder Pseudohalogenid-Ion ist.

5. Verfahren nach Anspruch 1, dadurch gekennzeichnet, daß einer der beiden Bindungspartner an einen festen Träger immobilisiert vorliegt.

6. Verfahren nach Anspruch 1, dadurch gekennzeichnet, daß der Antikörper oder das Antigen an einen optischen Lichtwellenleiter, oder am Ende eines faseroptischen Lichtleiters immobilisiert vorliegt und die Fluoreszenzlöschung über den Lichtwellenleiter oder den faseroptischen Lichtleiter verfolgt wird.

7. Verfahren nach Anspruch 1, dadurch gekennzeichnet, daß der Fluoreszenzimmunoassay in Serum, Plasma oder Vollblut durchgeführt wird, und daß der Löscher ein natürlicher Bestandteil des Serums, Plasmas oder Vollbluts ist.

Fig. 1

Ag

Ab

1a

Ab/Ag

1b

Fig. 2

Ag

Ab

2 a

Ab/Ag

2 b

Fig. 3

Ag

Ab

3 a

Ab/Ag

3 b

Fig. 4

Fig. 5

Fig. 6